# EUROPEAN PATENT APPLICATION

(11) **EP 4 728 983 A1**
(43) Date of publication of application: **22.04.2026**
(21) Application number: 24207479.7
(22) Date of filing: 18.10.2024
(51) Int. Cl.: A61B 5/145, A61B 5/00

(54) **MINIMALLY INVASIVE WEARABLE WITH INTEGRATED BISTABLE COMPLIANT APPLICATOR**

(71) Applicant: Roche Diabetes Care GmbH, 68305 Mannheim (DE)
(72) Inventor: SLIOZBERG, Kirill, 68305 Mannheim (DE)
(74) Representative: Grünecker Patent- und Rechtsanwälte PartG mbB

(57) **Abstract**

The invention relates to a medical device (100), in particular a wearable medical device (100), for detecting at least one analyte in a body fluid, the medical device (100) comprising: a housing (101) comprising: an analyte sensor (105) having an insertable portion (111) adapted for at least partially being insertable into a body tissue of a user; an inserter (113) comprising a bistable insertion mechanism (112), wherein the bistable mechanism (112) comprises a first stable state (112a) at a first position (115) and a second stable state (112b) at a second position (114), wherein in the first position (115) of the bistable insertion mechanism (112), the analyte sensor (105) is positioned in a retracted position (105a), and wherein in the second position (114) of the bistable insertion mechanism (112), the analyte sensor (105) is positioned in an advanced position (105b), and wherein the bistable insertion mechanism (112) is configured for advancing the analyte sensor (105) from the retracted position (105a) to the advanced position (105b) during a transition from the bistable insertion mechanism (112) from the first position (115) to the second position (114), whereby the insertable portion (111) of the analyte sensor (105) can be inserted into a body tissue of a user at least partially.

## Description

### Technical field

The invention relates to a medical device for detecting at least one analyte in a body fluid, to a method of using said medical device and to a method for assembling said medical device.

The device and methods according to the present invention may be used for long-term monitoring of an analyte concentration in a body fluid, such as for long-term monitoring of a blood glucose level or of the concentration of one or more other types of analytes in a body fluid. The invention may be applied in the field of home care as well as in the field of professional care, such as in hospitals. Other applications are also feasible.

### State of the art

Current wearable medical devices that provide long-term monitoring of an analyte concentration in a body fluid, such as continuous glucose monitoring (CGM) systems, are challenging to operate.

For example, current mechanism for applying wearable CGM devices, wherein an analyte sensor is at least partially inserted into a body tissue of a user, are complex, intricate and difficult to operate for the user.

Furthermore, the complexity of current mechanisms for applying wearable CGM devices also requires difficult, cumbersome and costly manufacturing steps during the assembly of current wearable CGM devices.

### Problem

It is therefore an objective of the present invention to improve a medical device, in particular a wearable medical device, for detecting at least one analyte in a body fluid.

In particular, it is an objective to simplify the constructional design of said medical device, and to increase the robustness, the handling and the user-friendliness of the medical device.

Furthermore, it is an objective to facilitate and simplify the assembly and use of a medical device comprising an analyte sensor.

### Solution

Said objectives are achieved by the subject-matter of the independent claims. Advantageous embodiments and further developments are the subject-matter of the dependent claims.

Further advantageous embodiments and further developments are disclosed throughout the specification.

For example, a medical device, in particular a wearable medical device, for detecting at least one analyte in a body fluid, may comprise:
- a housing, wherein said housing may comprise or accommodate:
   ∘ an analyte sensor having an insertable portion adapted for at least partially being insertable into a body tissue of a user, and
   ∘ an inserter comprising a bistable insertion mechanism, wherein the bistable mechanism comprises a first stable state at a first position and a second stable state at a second position.

In the first position of the bistable insertion mechanism, the analyte sensor can be positioned in a retracted position. In the second position of the bistable insertion mechanism, the analyte sensor can be positioned in an advanced position.

Said bistable insertion mechanism can be configured for advancing the analyte sensor from the retracted position to the advanced position during a transition from the bistable insertion mechanism from the first position to the second position, whereby the insertable portion of the analyte sensor can be inserted into a body tissue of a user at least partially, and wherein in the second stable state at the second position of the bistable insertion mechanism, the analyte sensor can be retained in the advanced position.

In other words, the bistable insertion mechanism can drive and guide the analyte sensor such that the insertable portion of the analyte sensor can at least partially be inserted into a body tissue of the user.

Said transition from the first stable state to the second stable state of the bistable insertion mechanism, i.e. the possible transition from the first position to the second position of the bistable insertion mechanism, can be reversible or irreversible.

During the possible transition from the first position to the second position of the bistable insertion mechanism, the bistable insertion mechanism may, for example, pass a snap-through point, representing an unstable intermediate state that the mechanism passes through during the transition.

The housing may comprise an opening or aperture that can receive the analyte sensor in said advanced position of the analyte sensor, i.e. when the bistable insertion mechanism is in said second stable state in said second position, to facilitate the at least partial insertion of the insertable portion of the analyte sensor into a body tissue of a user.

Herein, the terms first position and second position of the bistable insertion mechanism may also refer to a first configuration and to a second configuration of the bistable insertion mechanism.

The term "body fluid" generally may refer to a fluid which typically is present in a body or body tissue of the user or the patient and/or which may be produced by the body of the user or the patient. As an example for body tissue, interstitial tissue may be named. Thus, as an example, the body fluid may be selected from the group consisting of blood and interstitial fluid. However, additionally or alternatively, one or more other types of body fluids may be used, such as saliva, tear fluid, urine or other body fluids. During detection of the at least one analyte, the body fluid may be present within the body or body tissue. Thus, specifically, the analyte sensor may be configured for detecting at least one analyte in a body tissue.

As further used herein, the term "analyte" may refer to an arbitrary element, component or compound which may be present in the body fluid and the presence and/or the concentration of which may be of interest for the user, the patient or medical staff such as a medical doctor. Particularly, the analyte may be or may comprise an arbitrary chemical substance or chemical compound which may take part in the metabolism of the user or the patient, such as at least one metabolite.

As an example, the at least one analyte may be selected from the group consisting of glucose, cholesterol, triglycerides, lactate. Additionally or alternatively, however, other types of analytes may be used and/or any combination of analytes may be determined. The detection of the at least one analyte specifically may be an analyte-specific detection.

The medical device can specifically be configured as a continuous analyte monitoring system, e.g. as a continuous glucose monitoring (CGM) system.

The inserter may also be referred to as applicator and the bistable insertion mechanism may also be referred to as bistable application mechanism.

The above and herein described inserter comprising the bistable insertion mechanism may be configured such that the transition from the bistable insertion mechanism from the first position to the second position can occur along a central line of the housing, e.g. along a central axis or along a symmetry axis of the housing.

Said analyte sensor may comprise an array of microneedle analyte sensors or said analyte sensor can be an array of microneedle analyte sensors.

As indicated above, the medical device can inter alia be a wearable medical device that the user can wear on his body.

For example, the medical device can be a patch-type device, wherein at least some part of the housing can be realized as a patch unit that is placeable and wearable on the skin of the user.

The above and herein described medical device provides a simplified, efficient and more robust design for a medical device, in particular for a wearable medical device, for detecting at least one analyte in a body fluid.

In particular, the herein proposed medical device provides a simplified design for the insertion of an analyte sensor into the body tissue of a user. It improves the ease of operation for a user of the medical device, in particular regarding the at least partial insertion of the analyte sensor into the body tissue of the user. It allows a more effective, efficient and precise insertion of the analyte sensor in the body tissue of a user, thereby minimizing any potential pain or discomfort for the user during insertion and after.

Surprisingly, it has been found that the above and herein exemplary described design of the medical device in particular improves significantly the accuracy, efficiency and easy with which an array of microneedle analyte sensors can be inserted at least partially into the body tissue of the user.

The analyte sensor can be configured to be movable and displaceable within the housing, i.e. the analyte sensor can be arranged displaceable in the housing of the medical device.

The analyte sensor, e.g. said array of microneedle analyte sensors, can be directly coupled to the bistable insertion mechanism. For example, the analyte sensor, e.g. said array of microneedle analyte sensors, can be detachably coupled or can be detachably connected to the bistable insertion mechanism.

Alternatively, it is also possible that the analyte sensor, e.g. said array of microneedle analyte sensors, in the retracted position has no physical contact with the bistable insertion mechanism.

Stated differently, the analyte sensor, e.g. said array of microneedle analyte sensors, may be positioned or arranged such that only during the possible transition from the first position to the second position of the bistable insertion mechanism, the bistable insertion mechanism establishes a physical contact with the analyte sensor to displace, drive and guide the analyte sensor, such that the insertable portion of the analyte sensor can be inserted into a body tissue of a user at least partially.

The above and herein exemplary described medical device does not require any intricate or complicated insertion mechanisms. For example, the herein described bistable insertion mechanism requires neither any biased or preloaded components, e.g. springs, nor any retaining components, e.g. retaining hooks, thereby significantly simplifying the construction and use of the medical device. The herein described bistable insertion mechanism also does not require the use of expensive and more difficult to process materials, e.g. metals.

For example, the material of the above and herein exemplary described housing of the medical device and/or the material of the above and herein exemplary described bistable insertion mechanism can be comprised largely, mainly or fully of a plastic material or of a combination of different plastic materials.

For example, all modern plastics or their compositions could potentially be used, such as: polyethylene (including high-density polyethylene), polypropylene, polystyrene, polyethylene terephthalate, polyvinyl chloride, polycarbonate or acrylonitrile butadiene styrene.

The above and herein exemplarily described bistable insertion mechanism can be a compliant bistable insertion mechanism.

Said exemplary compliant bistable insertion mechanism can rely on elastic deformation of some of its components to transfer force and motion onto the analyte sensor.

Possible exemplary components of the compliant bistable insertion mechanism can be configured for bending, flexing, twisting, or stretching in a controlled manner to achieve a desired motion or force transmission onto the analyte sensor.

Again, the possible use of a compliant bistable insertion mechanism can further simplify the design of the medical device, inter alia allowing for the use of fewer parts as compared to current designs.

The above and herein exemplarily described inserter comprising the bistable insertion mechanism can be integrated into the housing of the medical device.

This can further simplify the design of the medical device and also can provide a more compact and easier to handle medical device, in particular a more compact, lighter and easier to handle wearable medical device. Stated differently, the above and herein described design can provide an improved minimally invasive medical device, in particular, an improved minimally invasive wearable CGM patch device that is easier to use and more comfortable to wear for a user.

As previously indicated, the housing of the medical device may comprise an opening or aperture that can receive the analyte sensor in said advanced position of the analyte sensor, i.e. when the bistable insertion mechanism is in said second stable state in said second position, to facilitate the at least partial insertion of the insertable portion of the analyte sensor into a body tissue of a user.

In particular, the above and herein exemplary described medical device may comprise a housing, wherein said housing can comprise a top part and a base part. Said exemplary base part of the housing may comprise an opening or aperture, e.g. a through-opening or through-hole, wherein said opening or aperture can be configured for receiving the analyte sensor in the advanced position, i.e. when the bistable insertion mechanism is in said second stable state in said second position, to facilitate the at least partial insertion of the insertable portion of the analyte sensor into a body tissue of a user.

The inserter comprising the bistable insertion mechanism can be integrated partially or fully in the top part of the housing.

Again this can allow the provision of a more compact and easier to handle medical device, in particular a more compact and easier to handle wearable medical device.

For example, the bistable insertion mechanism can be fully integrated into said exemplary top part of the housing, i.e. the bistable insertion mechanism can be an integral component of the top part or the top part can be the inserter, i.e. can be the bistable insertion mechanism.

For example, said bistable insertion mechanism that can be integrated into the top part of the housing or that can be the top part of the housing of the medical device, can be formed by a shuttle, at least one compliant segment and at least one frame segment, wherein the shuttle can be coupled to the at least one frame segment via the at least one compliant segment.

The analyte sensor can be directly coupled to the shuttle. However, as previously indicated, it is also possible that in the retracted position, the analyte sensor has no physical contact with the shuttle and only comes into physical contact during the transition of the bistable insertion mechanism from the first position to the second position of the bistable insertion mechanism.

The at least one frame segment can be rigid, i.e. not flexible, thereby providing a rigid reference frame that can constrain the possible movement of the bistable insertion mechanism, i.e. the displace movement of the shuttle, and thereby constraining and guiding the possible movement of the analyte sensor from its retracted position to its advanced position.

Said at least one frame segment can be directly coupled to or mounted to said exemplary base part of the housing.

The at least one compliant segment can be elastically deformed, e.g. bent or flexed, in a controlled way, when a force, e.g. a pushing force, is applied to the shuttle of the bistable mechanism, thereby allowing imparting a controlled linear displacement motion onto the analyte sensor from its retracted position to its advanced position.

The bistable insertion mechanism can be configured such for activating the bistable insertion mechanism by applying a force, e.g. a pushing force, for example, applying a force, e.g. a pushing force to the shuttle,

Said force, e.g. said pushing force, applied to the bistable insertion mechanism, e.g. to the shuttle, can be required to be a force greater than a predetermined force threshold, in order to overcome the first critical point of the bistable insertion mechanism and to initiate the transition from the bistable insertion mechanism from the first stable state to the second stable state of the bistable insertion mechanism.

When applying said force, e.g. said pushing force, on the bistable insertion mechanism, e.g. on the shuttle, for example, when applying a force, e.g. a pushing force, greater than said predetermined force threshold, in order to overcome the first critical point of the bistable insertion mechanism, a momentum can be obtained which can drive the analyte sensor via the shuttle to the advanced position of the analyte sensor.

For example, said predetermined force threshold can be around 0.5 N (newton) or higher. An exemplary pushing force could then exemplary lie in a range of 0.5 newton to 5 newtons or more.

The obtained momentum can be sufficient to allow the protrusion of the user's skin by the analyte sensor, thereby allowing at least partially inserting the insertable portion of the analyte sensor into the body tissue of the user.

Said force, e.g. said pushing force, can be applied parallel to or along the previously mentioned central line or central axis of the housing medical device which can coincide with the central line or central axis of the bistable insertion mechanism, in particular with the central line or central axis of the shuttle.

Said central line or central axis of the bistable insertion mechanism can be a symmetry axis of the bistable insertion mechanism.

This can further improve the accuracy and control over the linear displacement movement of the analyte sensor via the shuttle.

The shape of the bistable insertion mechanism can be fully or at least partially symmetric with respect to said central line or central axis of the medical device. This can further facilitate obtaining an accurate linear displacement motion of the shuttle and thereby facilitating obtaining a more accurate linear displacement motion of the analyte sensor into its advanced position

The exemplary top part comprising the inserter with the bistable insertion mechanism can be a single component. Said single component can be made from a plastic material, e.g. can be produced by injection molding. However, it is also conceivable that the top part and also other parts of the medical device can be produced by additive manufacturing or 3D printing.

Such a design further contributes to simplifying construction and manufacturing of the medical device and is more cost effective as compared to current medical devices.

As indicated above, the inserter comprising the bistable insertion mechanism can be integrated into the top part of the housing. In other words, the shuttle, the at least one compliant segment and the at least one frame segment can be understood as being formed by different zones or different segments or different sections or different regions or different parts of the top part of the housing.

This can inter alia be achieved by the top part comprising zones or segments or parts or regions or sections of varying thickness and/or of varying shapes or varying geometries.

In addition, it is possible that the bistable insertion mechanism comprises joints or joint-like components arranged between the at least one compliant segment, the shuttle and the at least one frame segment.

Said possible joints can be again integrated into the top part of the housing. Said possible joints can act as pivot points that allow rotational and translational movement.

Said possible joints can contribute to improving control and precision of the movement of the shuttle, thereby improving the control and accuracy with which the analyte sensor can be moved from the retracted position to the advanced position, and thereby improving the accuracy and control with which the insertable portion of the analyte sensor can be inserted into a body tissue of a user at least partially.

In particular, said possible joints can facilitate maintaining a movement of the analyte sensor that is plane-parallel to the user's skin surface, thereby again improving the accuracy and control of the insertion of the analyte sensor.

Said possible joints can again be realized by segments or sections or regions or parts of varying thickness and/or of varying shapes or varying geometries of the top part of the housing.

For example, said possible joints can be realized by segments or sections or regions or parts of the top part of the housing being thinner than neighboring segments or neighboring sections or neighboring regions or neighboring parts of the top part of the housing.

Alternatively, said possible joints could be realized by segments or sections or regions or parts of the top part of the housing comprising a more elastic material than neighboring segments or neighboring sections or neighboring regions or neighboring parts of the top part of the housing.

The shape or overall shape of the top part of the housing and/or the shape or overall shape of the inserter comprising the bistable insertion mechanism can be a hood, e.g. a rectangular or box-shaped hood, or a dome, e.g. a spherical or ellipsoidal dome, in particular a half-spherical or half-ellipsoidal shaped dome or a spherical cap or ellipsoidal cap shaped dome.

In particular, the top part of the housing comprising the bistable insertion mechanism can, when connected to the base part of the housing, form a cavity that can accommodate all other parts of the medical device, e.g. the analyte sensor, electronic components, and a power source, e.g. a battery.

In particular, the top part of the housing comprising the bistable insertion mechanism can have the shape of a spherical cap, having a small circle as its geometrical base. Alternatively, the top part of the housing comprising the bistable insertion mechanism can have the shape of an ellipsoidal cap, having an ellipse as its geometrical base.

The above discussed possible components, e.g. shuttle, at least one compliant segment, at least one frame segment and possible joints, of the bistable insertion mechanism can then be defined or formed by different zones of the spherical cap-shaped or of ellipsoidal cap-shaped top part of the housing, wherein the shuttle can be arranged centrally, i.e. can be formed by a segment or zone around and including the top of the spherical cap or of the ellipsoidal cap.

The base part of the housing of the above and herein exemplary described medical device can be a flat base, with a flat top surface and a flat bottom surface, wherein the flat bottom surface of the base part is facing towards the skin of the user, when the medical device is worn by a user.

The base part can be directly or indirectly attached to the skin of the user.

The base part of the housing of the medical device can be mounted to the top part of the housing of the medical device reversibly or irreversibly. For example, the base part of the housing can be mounted to the top part of the housing via a glue connection or a press-fit connection or via a friction-fit connection or via a form-fit connection, or via easily releasable mechanical connections, e.g. a snap-fit connection or screw or bolt connections.

The base part of the housing, e.g. the bottom surface, can comprises at least one adhesive element or adhesive layer, in particular a double-sided adhesive element or double-sided adhesive layer, e.g. a plaster or adhesive strip or adhesive ring, which can be configured for attaching the housing of the medical device to the skin of the user.

Said at least one adhesive element, aside from being configured for attaching the base part of the housing to the skin of the user, can further be configured for providing a sealing for the housing.

Alternatively or in addition, said at least one adhesive element can be configured for providing a fixation of the analyte sensor in the advanced position of the analyte sensor, e.g. it can be configured for providing a support at or within the opening or aperture of the base part that can receive the analyte sensor in the advanced position, thereby further facilitating retaining the analyte sensor in the advanced position and/or on the skin of a user in a well-defined, precise and controlled manner.

Said at least one adhesive element can be configured for covering the bottom surface of the base part of the housing at least partially or fully, including the opening or aperture of the base part that can receive the analyte sensor in the advanced position.

In the case of the opening or aperture of the base part that can receive the analyte sensor in the advanced position being also fully or partially covered by the at least one adhesive element, the at least one adhesive element can be pierced or penetrated by the analyte sensor when the analyte sensor transitions into the advanced position, wherein the analyte sensor is driven and guided by the bistable insertion mechanism.

Alternatively, the at least one adhesive element can be configured to surround the opening or aperture of the base part, e.g. the at least one adhesive element can have the shape of a circular or an elliptical ring or a rectangular ring.

Alternatively, or in addition, the at least one adhesive element, e.g. said double-sided adhesive element, or a further adhesive element, e.g. said double-sided adhesive element, may be attached or coupled to the analyte sensor, e.g. to the array of microneedle analyte sensors.

This can further improve facilitating retaining the analyte sensor in the advanced position in a well-defined, precise and controlled manner and/or on the skin of a user.

The above and herein exemplary described medical device may further comprise a first printed circuit board assembly and a second printed circuit board assembly, wherein the first printed circuit board assembly can comprise at least one electronic component, and wherein the second printed circuit board assembly can comprise at least one electronic component. Said possible first printed circuit board assembly and said possible second printed circuit board assembly may also be referred to as first flat structured conductor and as second flat structured conductor.

The first printed circuit board assembly and the possible optional second printed circuit board assembly can each comprise a plurality of conductors, e.g. conductive traces or tracks or paths or inter-integrated circuit (IIC) components, for electrically interconnecting the electronic components on the respective printed circuit board assembly.

For example, the first printed circuit board assembly may be mounted to the top surface of the base part of the housing of the medical device. For example, said first printed circuit board assembly can be mounted to the top surface of the base part via heat-stacking or hot-stacking or can be fixed or mounted between top surface of the base part and the top part of the housing of the medical device during assembly.

Furthermore, said first printed circuit board assembly may at least partially or fully surround the opening or aperture of the base part of the housing of the medical device.

The possible electronic components of said first printed circuit board assembly may inter alia include a power source, e.g. battery, as well as one or more digital processors or microcontrollers, and/or transceiver elements for wireless radio frequency communications, e.g. via Bluetooth or near-field communication (NFC) protocols.

Said possible transceiver elements can inter alia be used for initialization of the medical device and/or for communication with external devices.

Said first printed circuit board assembly may also be referred to as main printed circuit board assembly.

Alternatively or in addition, the analyte sensor may be coupled, in particular electronically coupled or operably connected, to said possible second printed circuit board assembly. Furthermore, the analyte sensor can be mechanically coupled to the possible second printed circuit board assembly.

Said possible optional second printed circuit board assembly may also be referred to as secondary printed circuit board assembly.

The first printed circuit board assembly and the second printed circuit board assembly can be electrically connected to each other via an optional cable, e.g. a flat flexible cable.

However, it is also possible that the analyte sensor is directly electrically connected to the first printed circuit board assembly via an optional cable, e.g. a flat flexible cable, i.e. the second printed circuit board assembly is entirely optional.

This possible cable does not hinder the displacement movement of the analyte sensor from its retracted position to its advanced position.

Said cable, e.g. said flat flexible cable, may also comprise further electronic components, e.g. antenna elements, e.g. transceiver elements for wireless radio frequency communications, e.g. via Bluetooth or near-field communication (NFC) protocols. In other words, antenna elements, e.g. transceiver elements for wireless radio frequency communications, e.g. via Bluetooth or near-field communication (NFC) protocols can be placed on the first printed circuit board assembly and/or the second printed circuit board assembly and/or the optional cable connecting the first printed circuit board assembly and the second printed circuit board assembly.

The possible electronic components of said optional second printed circuit board assembly may inter alia comprise one or more analog front-end and/or analog-to-digital/digital-to-analog converter (AD/DA converter). These components may be provided as discrete components or integrated into a single component in an application-specific integrated circuit (ASIC). In the case of using discrete analog front-end components, these can include, among others, operational amplifiers (OPAMPs), resistors, capacitors, etc.

Said converters can inter alia allow minimizing or reducing the number of required conductors between the second printed circuit board assembly and the first printed circuit board assembly and can also minimize signal noise.

Alternatively or in addition, the first printed circuit board assembly and the second printed circuit board assembly may each comprise one or more conductive electrical contact areas that are configured for establishing an electrical connection between the first printed circuit board assembly and the second printed circuit board assembly, when the analyte sensor is in the advanced position. This also allows establishing an electrical connection between the first printed circuit board assembly and the analyte sensor.

For example, when said shuttle of the bistable insertion mechanism is displaced during the transition of the bistable insertion mechanism from its first stable state to its second stable state, the optional second printed circuit board assembly can be displaced together with the analyte sensor from a retracted position to an advanced position.

In this advanced position of the second printed circuit board assembly, an electrical connection between the first printed circuit board assembly and the second printed circuit board assembly can then be established via said electrical contact areas.

In other words, the above mentioned cable is only a possible option for establishing an electrical connection between the first printed circuit board assembly and the optional second printed circuit board assembly and/or between the first printed circuit board assembly and the analyte sensor.

The possibility of not requiring a cable for connecting the first printed circuit board assembly and the second printed circuit board assembly again allows for the realization of a more compact medical device and also can significantly reduce manufacturing efforts and costs.

Said possible one or conductive electrical contact areas may, for example, be implemented as contact pads, having flat contact surfaces. The shape of the contact surfaces of electrical contact areas can, for example, be rectangular, circular or elliptical, curved, e.g. banana shaped. Other shapes are possible too.

The medical device can be configured for being electronically activated or initialized, when said possible electrical connection between the first printed circuit board assembly and the second printed circuit board assembly is established via said possible electrical contact areas, when the analyte sensor is in the advanced position.

At least some or all of the one or more electrical contact areas may comprise an electrically conductive adhesive tape or may be implemented as electrically conductive adhesive tape(s), e.g. electrically conductive adhesive tape pads.

Herein and thereafter, the terms electrically conductive adhesive tape(s) or electrically conductive adhesive tape pad(s) may also refer to electrically conductive adhesive transfer tape(s) or electrically conductive adhesive transfer tape pad(s).

In particular, said electrically conductive adhesive tapes or said electrically conductive adhesive tape pads can be isotropic electrically conductive adhesive tapes or isotropic electrically conductive adhesive tape pads, i.e. having a high electrically conductivity (e.g. less than 0.3 ohms) in all directions.

It is also conceivable that the electrically conductive adhesive tapes or electrically conductive adhesive tape pads could have the following contact resistances for different substrates, based on four-wire (Kelvin probe) resistance measurements using a 1.0 × 1.0 inch square of tape and a roughened, cleaned substrate surface: less than 2.5 ohms for aluminum/aluminum, less than 2.0 ohms for aluminum/stainless steel, less than 1.0 ohm for copper/stainless steel, and less than 0.5 ohms for copper/copper.

In addition to establishing an electrical connection, said possible electrically conductive adhesive tape(s) can also establish a mechanical bond between the first printed circuit board assembly and the second printed circuit board assembly, when the analyte sensor is in the advanced position, thereby further improving accuracy and control over the placement of the analyte sensor in its advanced position, and thereby further improving accuracy and control of the insertion of the insertable portion of the analyte sensor into a body tissue of a user.

For example, the possible second printed circuit board assembly may comprise two or four electrical contact areas, e.g. contact pads, and the first printed circuit board assembly may comprise two or four correspondingly spatially arranged electrical contact areas, e.g. contact pads.

Other numbers of electrical contact areas, e.g. contact pads, are possible too for providing sufficient electrical power and digital communication lines, e.g. IIC, components, to the second printed circuit board assembly.

As previously indicated, when an electrical connection between the electrical contact areas, e.g. contact pads, of the first printed circuit board assembly and the electrical contact areas, e.g. contact pads, of the second printed circuit board assembly is established, e.g. when the analyte sensor is in the advanced position, the electronic components of the first printed circuit board assembly and the second printed circuit board assembly can be configured for detecting said established electrical connection and for activating the medical device.

However, it is also conceivable that the activation of the medical device is triggered by receiving an external signal, e.g. via a Bluetooth or NFC protocol.

Said electrical contact areas, e.g. contact pads, may be arranged in circular manner on the first printed circuit board assembly and/or the second printed circuit board assembly. For example, said electrical contact areas can be arranged such as to surround the opening or aperture of the above described base part of the housing of the medical device.

It is further possible that the first printed circuit board assembly and/or the second printed circuit board assembly comprise at least two electrical contact areas, e.g. contact pads, that are bridged, i.e. they are electrically connected, e.g. via jumpers or solder bridges.

Having at least two electrical contact areas, e.g. contact pads, bridged, can enable a low electrical resistance connection between said electrical contact areas. For example, this can enable a low electrical resistance connection between two electrical contact areas on the first printed circuit board assembly, which may be used for the triggering of the system activation.

Since the contact resistance is very low, such a connection could even be used for the connection to a power source, e.g. the battery of the first printed circuit board assembly. Hence there would be no need to provide the medical device with some deep sleep mode, but the medical device could be basically stored disconnected from the power source, e.g. the battery of the first printed circuit board assembly, until application or use of medical device by a user.

Herein, low electrical resistance or low contact resistance can be understood as referring to electrical resistance of less than a few ohm (Ω), e.g. the electrical resistance may lie in the range of milliohms (mΩ) to a few ohms (Ω).

Alternatively or in addition to implementing at least some or all of said electrical contact areas, contact pads, as electrically conductive adhesive tape(s) or electrically conductive adhesive tape pads, said electrically conductive adhesive tapes or said electrically conductive adhesive tape pads may be replaced by at least one anisotropic electrically conductive adhesive tape.

Stated differently, multiple electrically conductive adhesive tapes pads can be replaced a single continuous piece of anisotropic electrically conductive adhesive tape.

Said possible exemplary anisotropic electrically conductive adhesive tape can inter alia have the shape of a circular or an elliptical ring or a rectangular loop or a rectangular frame.

In other words the possible exemplary anisotropic electrically conductive adhesive tape, e.g. when attached to the first printed circuit board assembly, can be configured to surround the opening or aperture of the base part of the housing of the medical device.

In the possible case of being attached to the second printed circuit board assembly, the possible exemplary anisotropic electrically conductive adhesive tape can have a shape that can fill the opening or aperture of the base part of the housing.

Anisotropic electrically conductive adhesive tape can be characterized by having a high axial electric conductivity, e.g. along the thickness of the tape (e.g. less than 0.3 ohm for a tape surface area of 6 mm² and a tape thickness of 2 mil (50.8 µm)), and acting as electrical insulators in all other directions in the plane of the tape, e.g. in all lateral directions (e.g. 3.4×10^14 ohms per square).

Said anisotropic electrically conductive adhesive tape may comprise a matrix filled with conductive particles which allow interconnection between substrates through the adhesive thickness and that are spaced far enough apart to be electrically insulating in the plane of the adhesive tape.

Using a single piece of anisotropic electrically conductive adhesive tape instead of multiple electrically conductive adhesive tape pads can further simplify constructions and reduce manufacturing costs of the medical device.

Hence, for example, the first printed circuit board assembly and the second printed circuit board assembly may be electrically connected using a single continuous anisotropic electrically conductive adhesive tape, which can be mounted to the first printed circuit board assembly and/or to the second printed circuit board assembly.

Thus, two structured flat conductors (SFC), i.e. the first printed circuit board assembly and the second printed circuit board assembly, can be electrically connected using one single, continuous piece of anisotropic electrically conductive adhesive tape, thereby establishing electric contact only between opposite lying conductors without causing a short circuit between adjacent pairs of conductors.

In addition to establishing an electrical connection between the first printed circuit board assembly and the second printed circuit board assembly, the anisotropic electrically conductive adhesive tape can at the same time also act as a sealing element for protecting the interior of the medical device against humidity and dirt and as an additional mechanical fixator for fixing and supporting the analyte sensor in its advanced position.

However, it also possible to use both, i.e. both anisotropic electrically conductive adhesive tape(s) and isotropic electrically conductive adhesive tape(s) or isotropic electrically conductive adhesive tape pads, for establishing electrical connections between the first printed circuit board assembly and the second printed circuit board assembly and for establishing electrical connections among electronic components on the respective printed circuit board assemblies.

For example, it is possible that an anisotropic electrically conductive adhesive tape is stacked on top of one or more isotropic electrically conductive adhesive tapes or on top of one or more isotropic electrically conductive adhesive tape pads.

The medical device can further comprise additional sealing elements, in particular ring-like sealing elements, e.g. one or more circular gaskets, that can be configured for sealing the opening or aperture in the base part of the housing when the analyte sensor has been moved or displaced to its advanced position by the bistable insertion mechanism.

Said optional sealing elements can be combined with or coupled to said isotropic electrically conductive adhesive tape pads and/or to said anisotropic electrically conductive adhesive tape(s).

The above and herein exemplary described medical device may further comprise a removable layer covering the base part of the housing of the medical device, in particular the bottom surface of the base part of the housing of the medical device.

Said possible removable layer can comprise or be coupled to a gas-permeable membrane that can cover the opening or aperture of the base part of the housing of the medical device.

This possible optional gas-permeable membrane allows the application of gas diffusion sterilization processes to sterilize the interior of the medical device, including the analyte sensor, during the manufacturing of the medical device, since the sterilization gas can penetrate the gas-permeable membrane.

Said possible optional gas-permeable membrane may be directly integrated into the removable layer or may be attached to the removable layer, e.g. via gluing or sewing or thermal bonding or chemical bonding.

Said removable layer can seal the housing of the medical device before the first application or first use of the medical device by the user to protect the interior of the medical device, including the analyte sensor, from contamination, e.g. unwanted microorganisms such as bacteria or fungi.

Hence, after the possible mentioned gas diffusion sterilization, no further additional packaging of the medical device is needed to protect the interior of the medical device, including the analyte sensor, from contamination.

To facilitate the removal of the removable layer, the removable layer may comprise a latch that can protrude from the housing of the medical device and that can easily be grabbed by the user to peel off the removable layer before the first use of the medical device.

The optional removable layer can further act as a liner that can cover and protect the possible at least one adhesive element or adhesive layer at the bottom surface of the housing and which can be configured for attaching the housing of the medical device to the skin of the user. In other words, said possible removable layer can protect said adhesive element or adhesive layer at the bottom surface of the housing before use.

An exemplary method of using the above and herein exemplary described medical device by a user may comprise one, some or all of the following steps:
- providing the medical device, wherein the bistable insertion mechanism of the inserter is in the first stable state at the first position and in which the analyte sensor is positioned in the retracted position;
- placing the medical device against a skin layer of the user; and
- applying a force, e.g. a pushing force or a compressing force, to the bistable insertion mechanism to bring the bistable insertion mechanism into its second stable state in the second position, thereby advancing the analyte sensor from the retracted position to the advanced position to at least partially insert the insertable portion of the analyte sensor into a body tissue of the user.

The step of applying a force, e.g. a pushing force or a compressing force, to the bistable insertion mechanism can comprise applying a force, e.g. a pushing force or a compressing force, greater than a predetermined force threshold, in order to overcome the first critical point of the bistable insertion mechanism and to initiate the transition from the bistable insertion mechanism from the first stable state to the second stable state of the bistable insertion mechanism.

Furthermore, said force can be applied along a/the central line or along the central axis of the medical device. In particular, said force, e.g. said pushing force, can be applied parallel to or along the previously mentioned central line or central axis of the housing medical device which can coincide with the central line or central axis of the bistable insertion mechanism, in particular with the central line or central axis of a/the shuttle of the bistable insertion mechanism.

For completeness, it is further noted that the exemplary shuttle of the bistable insertion mechanism may include a cavity that provides space to fully or at least partially accommodate the electronic components of a possible second printed circuit board assembly.

An exemplary method for assembling an above and herein exemplary described medical device may comprise at least one, some or all of the following steps:
- providing a first part, e.g. the top part, of the housing comprising the inserter that comprises the bistable insertion mechanism;
- providing a second part, e.g. the base part, of the housing;
- mounting the first printed circuit board assembly comprising at least one electronic component to the second part of the housing; and
- securing the first part of the housing and the second part of the housing to each other.

The analyte sensor may be detachably mounted to the first part, e.g. the top part, of the housing before securing the first part of the housing and the second part of the housing to each other.

Also, all required electrical connections or all required electronic components may be configured and/or mounted before securing the first part of the housing and the second part of the housing to each other.

After the step securing the first part of the housing and the second part of the housing to each other, the housing of the medical device may be sealed. For example, the housing may be sealed by applying or attaching the previously described removable layer with or without the optional gas-permeable membrane.

In addition, after the step securing the first part of the housing and the second part of the housing to each other and/or after applying or attaching the previously described removable layer with or without the optional gas-permeable membrane, the medical device, in particular the interior of the medical device, can be sterilized, e.g. by gas sterilization and/or by radiation sterilization.

The following figures illustrate only exemplary some technical aspects to foster the technical understanding of some of the claimed features and of some of the above described features of the medical device, its use and its assembly.
**Fig. 1****:** Exemplary medical device
**Fig. 2****:** Exemplary medical device
**Fig. 3****:** Exemplary printed circuit board assemblies
**Fig. 4****:** Exemplary alternative medical device
**Fig. 5****:** Exemplary alternative printed circuit board assemblies

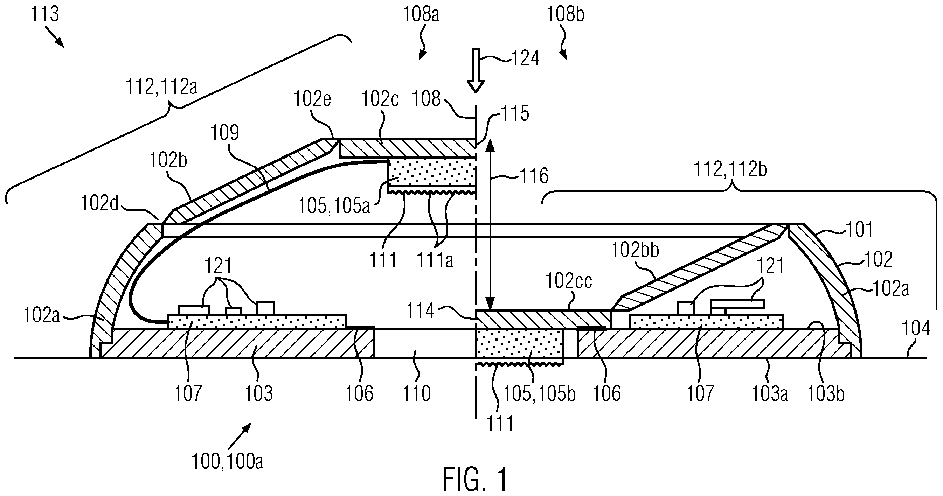

**Fig. 1** shows an exemplary medical device, in particular an exemplary wearable medical device 100, e.g. minimally invasive wearable CGM patch device, for detecting at least one analyte in a body fluid and that can comprise at least some or all of the above described exemplary device features.

**Fig. 1** shows an exemplary side view 100a of the exemplary medical device in two different exemplary states 108a, 108b that will be described further below.

The medical device 100 comprises an exemplary housing 101 that comprises an exemplary base part 103 and an exemplary top part 102.

The exemplary top part 102 has an exemplary shape of a spherical cap and the base part 103 exemplary can form the geometrical base of said spherical cap. As described above, other shapes of the housing, the top part and the base part are possible too.

The housing 101, in particular the top part 102 and/or the base part 103, can have a rotational symmetry; in particular, a rotational symmetry with respect to the exemplary central line or central axis 108 of the medical device.

The rotational symmetry with respect to the exemplary central axis 108 can be full or partial, e.g. can be symmetric only with respect to certain rotation angles around the axis 108.

The housing 101 further accommodates an exemplary analyte sensor 105 having an insertable portion 111 adapted for at least partially being insertable into a body tissue of a user. In the shown example, the analyte sensor 105 is implemented as comprising an exemplary array of microneedle analyte sensors 111a.

Stated differently, the exemplary analyte sensor 105 can have a plurality of insertable portions 111 realized by the plurality of microneedle analyte sensors 111a.

The medical device 100 further comprises an exemplary inserter 113 with which the insertable portion 111 of the analyte sensor can be inserted into a body tissue of a user at least partially.

The exemplary inserter 113 can comprise a bistable insertion mechanism 112, in particular, a compliant bistable insertion mechanism, which can be integrated into the housing 101.

In the shown example, the inserter 113 comprising the bistable insertion mechanism 112 is integrated in the top part 102 of the housing 101, wherein the bistable insertion mechanism is formed by an exemplary shuttle 102c, an exemplary compliant segment 102b and an exemplary frame segment 102a, wherein the shuttle 102c is coupled to the frame segment 102a via the compliant segment 102b, and wherein the compliant segment 102b is coupled to the shuttle 102c and to the frame segment 102a via the exemplary and optional joints 102d, 102e.

Due to the exemplary rotational symmetry of the top part 102 of the housing 101, the exemplary compliant segment 102b, the exemplary frame segment 102a, and the optional possible exemplary joints 102d, 102e can have a ring-like shape, whereas the centrally arranged exemplary shuttle 102c can have a spherical-cap shape or disc shape.

The exemplary frame segment 102a can be rigid, i.e. not flexible, and can be directly coupled to or mounted to the base part 103 of the housing 101. The exemplary frame segment 102a can be understood as a lateral wall fully or at least partially surrounding the base part 103 of the housing 101.

In the shown example, the analyte sensor 105 is directly coupled to the bistable insertion mechanism 112, i.e. is directly coupled to the shuttle 102c.

However, it is also possible that the analyte sensor 105 has no physical contact with the shuttle 102c, when the analyte sensor 105 is in the retracted position 105a and only comes into physical contact during the transition of the bistable insertion mechanism 112 from the first position 115 to the second position 114 of the bistable insertion mechanism 112.

The analyte sensor 105 can be electrically connected to an exemplary first printed circuit board assembly 107 via an exemplary optional cable 109, e.g. a flat flexible cable.

However, as indicated above and further below, other ways of electrically connecting the analyte sensor 105 to the exemplary first printed circuit board assembly 107 are possible too. For example, the analyte sensor may be mounted to a second printed circuit board assembly (not shown) and the first printed circuit board assembly 107 and the possible optional second printed circuit board assembly may be electrically connected via electrical contact areas on the printed circuit board assemblies.

The exemplary first printed circuit board assembly 107 can be mounted to the top surface 103b of the base part 103 and can comprise a plurality of electronic components 121, which can include a power source, e.g. a battery, and other further above discussed electronic components.

The exemplary first printed circuit board assembly 107 can partially or fully surround the exemplary opening 110, e.g. the exemplary central through-opening 110, of the base part 103 of the housing 101.

As previously mentioned, Fig. 1 shows two exemplary states 108a, 108b of the medical device 100, in particular two exemplary states 112a, 112b of the bistable insertion mechanism 112.

In the exemplary first state 108a of the medical device 100 shown on the left side, the bistable insertion mechanism 112 is in its exemplary first stable state 112a, wherein the bistable insertion mechanism 112 is at the exemplary first position 115, wherein said first position 115 is exemplary marked as corresponding to first position along the exemplary central line or central axis 108 of the medical device 100.

In this exemplary first stable state 112a of the bistable insertion mechanism 112, the analyte sensor 105 is in the exemplary retracted position 105a.

In the exemplary second state 108b of the medical device 100 shown on the right side, the bistable insertion mechanism 112 is in its exemplary second stable state 112b, wherein the bistable insertion mechanism 112 is at the exemplary second position 114, wherein said second position 114 is exemplary marked as corresponding to a second position along the exemplary central line or central axis 108 of the medical device 100 that is different from the first position.

In this exemplary second stable state 112b of the bistable insertion mechanism 112, the analyte sensor 105 is in the exemplary advanced position 105b and the shuttle 102c has been displaced along the central axis 108 to position 102cc.

In this exemplarily advanced position 105b of the analyte sensor 105, the insertable portion 111, e.g. the exemplary array of microneedle analyte sensors 111a, is exemplary partially inserted into body tissue of a user, e.g. by having penetrated the exemplary skin layer 104 of the user.

The exemplary displacement 116 of the analyte sensor 105 along the central axis 108 via the shuttle 102c of the bistable insertion mechanism 112 is caused by the transition of the bistable insertion mechanism 112 from its first stable state 112a at position 115 to its second stable state 112b at position 114.

This transition from the first stable state 112a of the bistable insertion mechanism 112 to its second stable state 112b can be initiated by applying a force, e.g. a pushing force, to the shuttle 102c and can displace the shuttle 102c along the exemplary central axis 108 to its exemplary displaced position 102cc.

In particular, said force can be applied parallel to or along the exemplary central axis 108, as exemplary indicated by the illustrated force vector 124.

Said force 124 can be required to be a force greater than a predetermined force threshold, in order to overcome the first critical point of the bistable insertion mechanism and to initiate the transition from the bistable insertion mechanism from the first stable state 112a to the second stable state 112b of the bistable insertion mechanism 112.

In this example, the applied force 124 is also orthogonal or substantially orthogonal to the bottom surface 103a of the base part 103 and the exemplary skin layer 104 of the user.

During this exemplary transition, the bistable insertion mechanism 112 can pass through a snap-through point that may also lie along the exemplary central axis 108.

As previously indicated, the base part 103 can be flat, with a flat bottom surface 103a and a flat top surface 103b, and can comprise an opening 110, an exemplary central through-opening 110 aligned with the exemplary central axis 108, for receiving the displaced shuttle 102cc together with the displaced analyte sensor 105 in its advanced position 105b, in which the analyte sensor 105 can be at least partially inserted into the body tissue of the user.

The bottom surface 103a of the base part 103 can be attached to the skin 104 of the user, e.g. via an adhesive tape (not shown), e.g. double-side adhesive tape.

The reference sign 106 marks an exemplary optional sealing element, e.g. a gasket, that is configured to seal the opening 110 in the base part 103.

The sealing element 106 can act as a sealing between the base part 103 and the shuttle 102c, when the shuttle 102c is in its displaced position 102cc, i.e. when the analyte sensor 105 has been displaced and guided by the shuttle 102c to its advanced position 105b.

For completeness, it is noted that the left side of Fig. 1 shows only a first half of the medical device 100 and the right side shows the other second half of the medical device, wherein the left side shows the first half of the medical device 100 with the bistable insertion mechanism 112 being in the first stable state 112a and with the right side showing the second half of the medical device 100 with the bistable insertion mechanism 112 being in the second stable state 112b.

It is emphasized again that the illustrated features of medical device 100 are exemplary only. For example, it is possible that instead of the single compliant segment 102b, the bistable insertion mechanism 112 may comprise a plurality of compliant segments that can couple the exemplary shuttle 102c to the exemplary frame segment 102a.

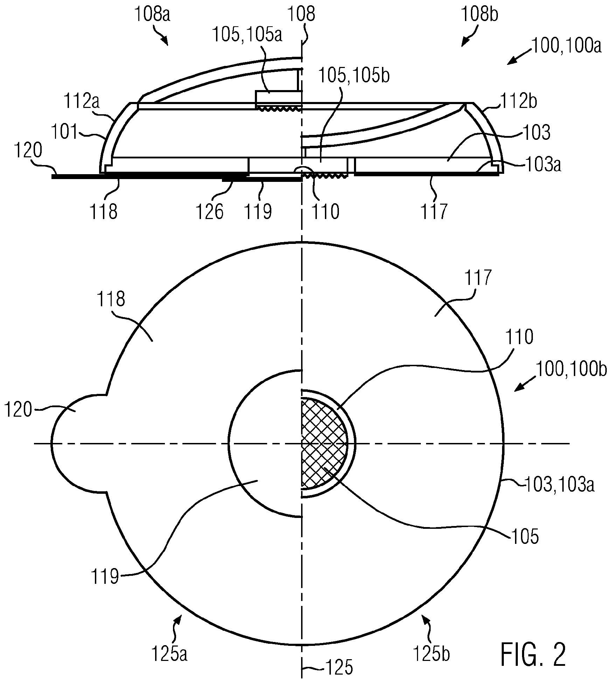

**Fig. 2** exemplary shows two different views of the medical device 100 of Fig. 1.

Fig. 2 shows a further simplified version of the side view 100a of the medical device 100 depicted in Fig. 1, showing again the two different states 108a, 108b of the medical device 100, with the two different stable states 112a, 112b of the bistable insertion mechanism of the medical device, and an exemplary view 100b onto the bottom surface 103a of the base part 103 of the housing of the medical device 100.

To avoid an unnecessary clutter of reference signs in the side view 100a, only a few of the reference signs from Fig. 1 also have been included in this side view 100a, including the reference signs for the bottom surface 103a of the base part 103, the reference sign for the central axis 108, the reference signs for the analyte sensor 105 in the retracted position 105a and in the advanced position 105b and the opening 110 in the base part 103 of the housing 101 of the medical device 100.

In addition to Fig. 1, Fig. 2 shows an exemplary and optional adhesive element 117, e.g. a double-sided adhesive element, as an exemplary adhesive layer that at least partially can cover the bottom surface 103a of the base part 103, and which can facilitate the attachment of the medical device 100 to the skin of a user.

Said adhesive element or adhesive layer 117 may surround the opening 110 in the base part 103 or can also cover the opening 110. In the latter exemplary case, said adhesive element or adhesive layer 117 can be configured to be pierceable by the analyte sensor 105 when it is displaced to its advanced position 105b.

Furthermore, Fig. 2 illustrates an exemplary optional removable layer 118 that can be attached to the adhesive element 117 and that further can include or that can be coupled to, an exemplary and optional gas-permeable membrane 119, wherein said gas-permeable membrane 119 can cover the opening 110 of the base part 103 of the housing 101 of the medical device 100.

The removable layer 118 can be removed by the user before the use of the medical device 100.

To facilitate the removal, the removable layer 118 may comprise a latch 120 that can protrude from the housing 101 of the medical device 100 and that can be easily grabbed by the user to peel off the removable layer 118 from the adhesive element 117 and/or from the bottom surface 103a of the base part 103 of the medical device 100.

The exemplary removable layer 118, the gas-permeable membrane 119 and the adhesive element 117 are shown from a different perspective in view 100b, which shows a view onto the bottom surface 103a of the base part 103 of the medical device 100.

The view 100b again is split into two halves 125a, 125b to better illustrate the exemplary possible stratification of the different possible layers 117, 118 and 119.

The exemplary radial axis 125 of base part 103 divides the medical device again into said exemplary halves 125a, 125b

The left half 125a of the view 100b shows the exemplary ring shape of the removable layer 118 with the exemplary circular disc shaped gas-permeable membrane 119 that is configured to cover the opening 110 of the base part 103 of the housing 101 of the medical device 100 and the exemplary optional latch 120 of the removable layer 118.

The right half 125b of the view 100b exemplary shows the exemplary ring-shaped adhesive layer 117 that can be located below the removable layer 118, i.e. between the removable layer 118 and the bottom surface 103a of the base part 103 of the housing 101 of the medical device 100.

The optional gas-permeable membrane 119 can be integrated into the removable layer 118 or can be coupled to or secured to the removable layer 118. In the shown example, the gas-permeable membrane 119 is larger than the size of the opening 110 and has an exemplary and optional overlap 126 with the removable layer 118. Said optional overlap 126 can facilitate the connection and attachment of the gas-permeable membrane 119 to the removable layer 118.

The right half 125b of the view 100b also exemplary shows the analyte sensor 105 of the medical device that can be seen through the opening 110 of the base part 103 of the housing 101 of the medical device 100.

It is noted that the illustrated shapes for all shown components is exemplary only. For example, instead of the exemplary circular shape of the analyte sensor 105, a rectangular shape or other shapes are possible too.

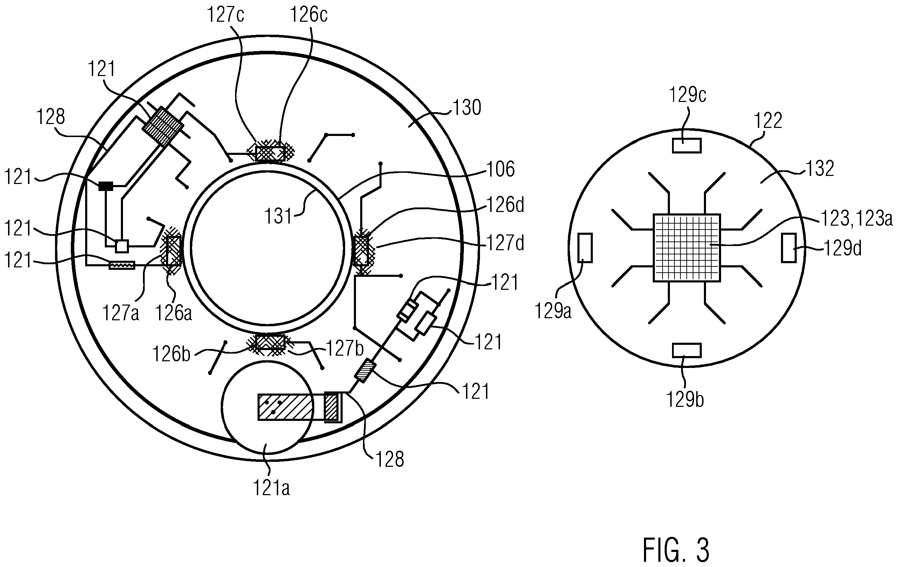

**Fig. 3** exemplary shows two possible exemplary printed circuit board assemblies; a first printed circuit board assembly 130 and a second printed circuit board assembly 122, wherein the second printed circuit board assembly 122 is coupled, in particular mechanically coupled and/or electrically coupled, to an exemplary analyte sensor 123 with an exemplary array 123a of microneedle analyte sensors.

The perspective of Fig. 3 shows top views of the surfaces of the two possible exemplary printed circuit board assemblies 130, 122 oriented such that, when the exemplary printed circuit board assemblies 130, 122 are mounted inside the housing of the medical device, e.g. inside the housing 101, the shown visible surfaces would face each other.

The analyte sensor 123 and exemplary array 123a of microneedle analyte sensors has an exemplary rectangular shape. However, it is also possible that the analyte sensor has another shape, e.g. a circular shape such as the analyte sensor 105 from Fig. 1 or Fig. 2.

Said exemplary printed circuit board assemblies could be implemented in any of the above and herein described medical devices.

For example, the exemplary first printed circuit board assembly 107 of the medical device 100 of Fig. 1 may be identical or similar to the first printed circuit board assembly 130 shown here.

However, unlike in the example of Fig. 1, the first printed circuit board assembly 130 shown here does not require any cable for establishing an electrical connection with the analyte sensor 123, since both exemplary printed circuit board assemblies are provided with exemplary electrical contact areas 126a, 126b, 126c, 126d, 129a, 129b, 129c, 129d that allow establishing an electrical connection between the first printed circuit board assembly 130 and the second printed circuit board assembly 122, when the analyte sensor 123 is in the advanced position, thereby also establishing an electrical connection between the first printed circuit board assembly 130 and the analyte sensor 123.

The exemplary first printed circuit board assembly 130 is exemplary provided with four exemplary electrical contact areas 126a, 126b, 126c, 126d that are exemplary uniformly distributed around the exemplary opening 131 of the first printed circuit board assembly 130, wherein the shape and size of the exemplary opening 131 can correspond to the opening in the base part of the medical device, e.g. the opening 110 of the base part 103 of the medical device 100 of Fig. 1 and Fig. 2.

Stated differently, the shape and size of the exemplary first printed circuit board assembly 130 can correspond to the shape and size of the exemplary base part 103 of the medical device 100 of Fig. 1 and Fig. 2. However, other shapes of the first printed circuit board assembly 130 are conceivable too.

The shown number and distribution of the exemplary electrical contact areas 126a, 126b, 126c, 126d are also exemplary only.

Furthermore, said exemplary electrical contact areas 126a, 126b, 126c, 126d are exemplary covered by electrically conductive adhesive transfer tapes or electrically conductive adhesive transfer tape pads 127a, 127b, 127c, 127d. Said tapes or pads can in particular be isotropic electrically conductive adhesive tapes or isotropic electrically conductive adhesive tape pads.

The rectangular shapes of the electrical contact areas 126a, 126b, 126c, 126d, 129a, 129b, 129c, 129d and of the exemplary electrically conductive adhesive tape pads 129a, 129b, 129c, 129d is exemplary only, i.e. other shapes are possible too.

Fig. 3 further exemplary shows exemplary electronic components 121 that are mounted on top of the first printed circuit board assembly 130, including an exemplary battery 121a. Furthermore, the first printed circuit board assembly 130 can comprise a plurality of conductors, e.g. conductive traces or tracks or paths or inter-integrated circuit (IIC) components, as exemplary marked with the reference sign 128.

Said exemplary electronic components 121 can comprise any of the further above described electronic components, e.g. digital processors or microcontrollers, and/or transceiver elements for wireless radio frequency communications, e.g. via Bluetooth or near-field communication (NFC) protocols.

The exemplary second printed circuit board assembly 122, to which the analyte sensor 123 can be mounted and electrically coupled, can comprise electrical contact areas 129a, 129b, 129c, 129d that can correspond to the electrical contact areas 126a, 126b, 126c, 126d of the first printed circuit board assembly 130. In other words, the number, distribution and spatial arrangement of the electrical contact areas 129a, 129b, 129c, 129d can correspond to the number, distribution and spatial arrangement of the electrical contact areas 126a, 126b, 126c, 126d. The spatial arrangement of the electrical contact areas 129a, 129b, 129c, 129d at the periphery of the second printed circuit board assembly 122 is exemplary.

Hence, when the analyte sensor 123 is in the advanced position, the electrical contact areas 126a, 126b, 126c, 126d of the first printed circuit board assembly 130 that are covered by the electrically conductive adhesive transfer tape pads 127a, 127b, 127c, 127d can come into direct physical contact with the electrical contact areas 129a, 129b, 129c, 129d of the second printed circuit board assembly 122, thereby allowing the establishment of an electrical connection between the first printed circuit board assembly 130 and the second printed circuit board assembly 122, including the analyte sensor 123.

In the shown example, only the electrical contact areas 126a, 126b, 126c, 126d of the first printed circuit board assembly 130 are covered by the electrically conductive adhesive transfer tape pads 127a, 127b, 127c, 127d.

However, it is also possible that electrically conductive adhesive transfer tape pads are only placed on the electrical contact areas 129a, 129b, 129c, 129d of the second printed circuit board assembly 122, or that some electrically conductive adhesive transfer tape pads are placed on some, e.g. half, e.g. two, of the electrical contact areas 126a, 126b, 126c, 126d of the first printed circuit board assembly 130 and that some electrically conductive adhesive transfer tape pads are placed on some, e.g. half, e.g. two, of the electrical contact areas 129a, 129b, 129c, 129d of the second printed circuit board assembly 122.

The exemplary circular shape and size of the exemplary second printed circuit board assembly 122 can correspond to the shape and size of the shuttle of bistable insertion mechanism of the medical device, e.g. to the shape and size of shuttle 102c of the bistable insertion mechanism 112 of the medical device 100 of Fig. 1 and Fig. 2.

The exemplary second printed circuit board assembly 122 can further comprise one or more electronic components, e.g. on the other side of the printed circuit board assembly 122 that is not visible in Fig. 3. The reference sign 132 exemplary marks possible exemplary conductors or conductive paths on the shown visible side of the second printed circuit board assembly 122.

For completeness, it is noted that reference sign 106 marks the exemplary optional sealing element, e.g. a gasket, that is configured to seal the opening 110 in the base part 103 of the medical device 100 of Fig. 1 and Fig. 2. Said sealing element can act as a sealing between the base part 103 and the shuttle 102c, when the shuttle 102c is in its displaced position 102cc, i.e. when the analyte sensor 105 has been displaced and guided by the shuttle 102c to its advanced position 105b.

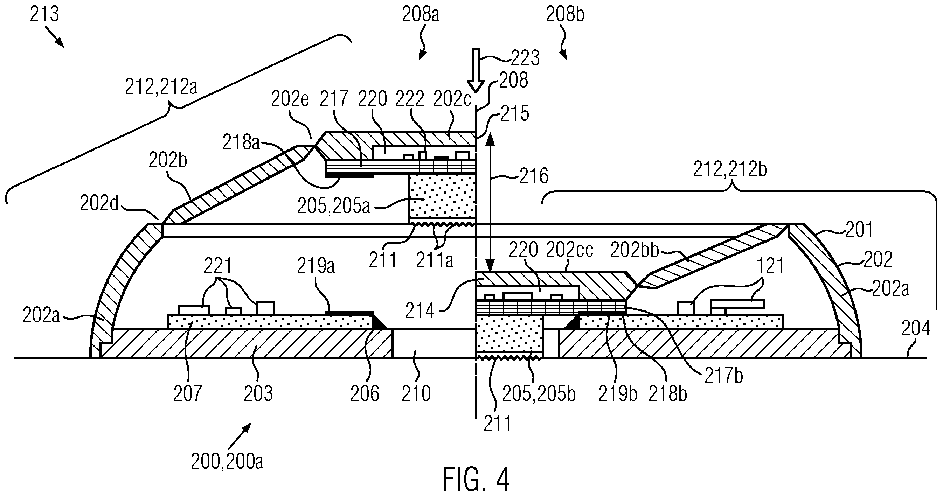

**Fig. 4** shows a medical device 200 that in most aspects is identical to the medical device 100 of Fig. 1 and Fig. 2.

Similar to Fig. 1, Fig. 4 shows a side view 200a of the medical device 200 in two different exemplary states 208a, 208b that can correspond to the states 108a, 108b described for the medical device 100 of Fig. 1 and Fig. 2.

The medical device 200 comprises an exemplary housing 201 that comprises an exemplary base part 203 and an exemplary top part 202 similar to the medical device 100.

The exemplary top part 202 has an exemplary shape of a spherical cap and the base part 203 exemplary can form the geometrical base of said spherical cap. As described above, other shapes of the housing, the top part and the base part are possible too.

The housing 201, in particular the top part 202 and/or the base part 203, can have a rotational symmetry; in particular, a rotational symmetry with respect to the exemplary central line or central axis 208 of the medical device.

The rotational symmetry with respect to the exemplary central axis 208 can be full or partial, e.g. can be symmetric only with respect to certain rotation angles around the axis 108.

The housing 201 further accommodates an exemplary analyte sensor 205 having an insertable portion 211 adapted for at least partially being insertable into a body tissue of a user.

In the shown example, the analyte sensor 205 is implemented as comprising an exemplary array of microneedle analyte sensors 211a. Said array of microneedle analyte sensors 211a can be identical or similar to the array of microneedle analyte sensors described in Fig. 1, Fig. 2 or Fig. 3.

Similar to the medical device 100, the medical device 200 comprises an exemplary inserter 213 with which the insertable portion 211 of the analyte sensor 205 can be inserted into a body tissue of a user at least partially.

The exemplary inserter 213 can comprise a bistable insertion mechanism 212; in particular, a compliant bistable insertion mechanism, which can be integrated into the housing 201 in a similar way as the inserter 113 of the medical device 100.

Similar to the bistable insertion mechanism 112 of medical device 100, the bistable insertion mechanism 212 of medical device 200 is formed by an exemplary shuttle 202c, an exemplary compliant segment 202b and an exemplary frame segment 202a, wherein the shuttle 202c is coupled to the frame segment 202a via the compliant segment 202b, and wherein the compliant segment 202b is coupled to the shuttle 202c and to the frame segment 202a via the exemplary and optional joints 202d, 202e.

The exemplary frame segment 202a can be rigid, i.e. not flexible, and can be directly coupled to or mounted to the base part 203 of the housing 201. The exemplary frame segment 202a can be understood as a lateral wall fully or at least partially surrounding the base part 203 of the housing 201.

However, unlike in the case of medical device 100, in the medical device 200 shown here, the analyte sensor 205 is not directly coupled to the shuttle 202c of the bistable insertion mechanism 212, but is first coupled, in particular mechanically coupled and/or electrically coupled, to the exemplary second printed circuit board assembly 217.

The exemplary second printed circuit board assembly 217 can then mechanically be coupled to the shuttle 202c of the bistable insertion mechanism 212, thereby indirectly coupling the analyte sensor 205 to the shuttle 202c such that the shuttle 202c can displace the analyte sensor 205 to its advanced position 205b when the bistable insertion mechanism 212 transitions from its first stable state 212a to its second stable state 212b. Since the exemplary second printed circuit board assembly 217 is coupled to the analyte sensor 205, said transition also displaces the exemplary second printed circuit board assembly 217 to an advanced position, i.e. to position 217b.

The exemplary second printed circuit board assembly 217 can be identical or similar to the second printed circuit board assembly 122 described in Fig. 3.

The exemplary second printed circuit board assembly 217 can comprise one or more electronic components 222. The exemplary shuttle 202c may comprise an exemplary cavity 220 that can provide space for fully or at least partially accommodating the one or more electronic components 222 of the second printed circuit board assembly 217.

The exemplary second printed circuit board assembly 217 and the exemplary first printed circuit board assembly 207 can be electrically connected to each other without the use of a cable in a manner described above, e.g. in the context of Fig. 3.

The second printed circuit board assembly 217 can have electrical contact areas, e.g. electrical contact area 218a, 218b, that can come into physical contact with corresponding electrical contact areas 219a, 219b of the first printed circuit board assembly 207 that is mounted to the base part 203 of the housing 201 of the medical device 200.

Similar to the exemplary electrical contact areas described in Fig. 3 for the printed circuit board assemblies 130, 122, the electrical contact areas of the printed circuit board assemblies 207, 217 can be covered by electrically conductive adhesive transfer tapes or electrically conductive adhesive transfer tape pads.

Said optional conductive adhesive transfer tapes or electrically conductive adhesive transfer tape pads can be placed either on the electrical contact areas of the first printed circuit board assembly 207 or on the second printed circuit board assembly 217 or partially on both, e.g. on half of the electrical contact areas of the first printed circuit board assembly 207 and on half of the electrical contact areas of the second printed circuit board assembly 217.

Similar to Fig. 3, the printed circuit board assemblies 207, 217 can each comprise corresponding four electrical contact areas or another number of matching electrical contact areas. In the shown example, only two exemplary electrical contact areas 218a, 218b, 219a, 219b on each printed circuit board assembly 207, 217 are shown.

Said physical contact and electrical connection between the printed circuit board assemblies 207, 217, thereby enabling also an electrical connection between the analyte sensor 205 and the first printed circuit board assembly 207 can be established when the bistable insertion mechanism 212 transitions from its first stable state 212a to its second stable state 212b.

As previously mentioned, Fig. 4 shows two exemplary states 208a, 208b of the medical device 200, in particular two exemplary states 212a, 212b of the bistable insertion mechanism 212.

In the exemplary first state 208a of the medical device 200 shown on the left side, the bistable insertion mechanism 212 is in its exemplary first stable state 212a, wherein the bistable insertion mechanism 212 is at the exemplary first position 215, wherein said first position 215 is exemplary marked as corresponding to first position along the exemplary central line or central axis 208 of the medical device 200.

In the exemplary second state 208b of the medical device 200 shown on the right side, the bistable insertion mechanism 212 is in its exemplary second stable state 212b, wherein the bistable insertion mechanism 212 is at the exemplary second position 214, wherein said second position 214 is exemplary marked as corresponding to a second position along the exemplary central line or central axis 208 of the medical device 200 that is different from the first position.

In this exemplary second stable state 212b of the bistable insertion mechanism 212, the analyte sensor 205 is in the exemplary advanced position 205b and the shuttle 202c has been displaced along the central axis 208 to position 202cc. The displacement of the shuttle 202c displaces both the analyte sensor 205 and the second printed circuit board assembly 217.

In this position 202cc of the shuttle 202c, a physical and electrical connection can be established between the printed circuit board assemblies 207, 217 via the electrical contact areas 218a, 218b, 219a, 219b of the printed circuit board assemblies 207, 217 that also come into physical contact when shuttle 202c is in position 202cc and the second printed circuit board assembly 217 is in position 217b, i.e. when the analyte sensor 205 is in the advanced position.

This way, also the analyte sensor 205 can electrically be connected to the first circuit board assembly 207.

In this exemplary advanced position 205b of the analyte sensor 205, the insertable portion 211, e.g. the exemplary array of microneedle analyte sensors 211a, is exemplary partially inserted into body tissue of a user, e.g. by having penetrated the exemplary skin layer 204 of the user.

The analyte sensor 205 can be maintained in said advanced position 205b by the bistable insertion mechanism 212 for the duration of use of the medical device 200.

The exemplary displacement 216 of the analyte sensor 205 along the central axis 208 via the shuttle 202c of the bistable insertion mechanism 212 can be caused by the transition of the bistable insertion mechanism 212 from its first stable state 212a at position 215 to its second stable state 212b at position 214.

This transition from the first stable state 212a of the bistable insertion mechanism 212 to its second stable state 212b can be initiated by applying a force, e.g. a pushing force, to the shuttle 202c and can displace the shuttle 202c along the exemplary central axis 208 to its exemplary displaced position 202cc.

In particular, said force can be applied parallel to or along the exemplary central axis 208, as exemplary indicted by the illustrated force vector 223.

Said force vector 223 can be required to be a force greater than a predetermined force threshold, in order to overcome the first critical point of the bistable insertion mechanism and to initiate the transition from the bistable insertion mechanism from the first stable state 212a to the second stable state 212b of the bistable insertion mechanism 212.

In this example, the applied force 223 is also orthogonal or substantially orthogonal to the bottom surface of the base part 203 and the exemplary skin layer 204 of the user.

During this exemplary transition, the bistable insertion mechanism 212 can pass through a snap-through point that may also lie along the exemplary central axis 208.

Similar to the medical device 100, the base part 203 of the medical device 200 can be flat, with a flat bottom surface and a flat top surface, and can comprise an opening 210.

This exemplary central through-opening 210 can be aligned with the exemplary central axis 208, for receiving the displaced shuttle 202cc together with the displaced analyte sensor 205 in its advanced position 205b, in which the analyte sensor 205 can be at least partially inserted into the body tissue of the user.

Similar to the exemplary first printed circuit board assembly 107 of medical device 100 and the exemplary first printed circuit board assembly 130 of Fig. 3, the exemplary first printed circuit board assembly 207 of medical device 200 also can comprise a plurality of electronic components, including inter alia a battery.

Furthermore, similar to the exemplary first printed circuit board assembly 107 of medical device 100 and the exemplary first printed circuit board assembly 130 of Fig. 3, the exemplary first printed circuit board assembly 207 of the medical device 200 can at least partially or fully surround the opening 210 of the base part 203 of the housing 201 of the medical device 200.

The reference sign 206 marks an exemplary optional sealing element, e.g. a gasket, that is configured to seal the opening 210 in the base part 203.

The sealing element 206 can act as a sealing between the base part 203 and the shuttle 202c, when the shuttle 202c is in its displaced position 202cc, i.e. when the analyte sensor 205 has been displaced and guided by the shuttle 202c to its advanced position 205b.

For completeness, it is noted that the left side of Fig. 4 shows only a first half of the medical device 200 and the right side shows the other second half of the medical device, wherein the left side shows the first half of the medical device 200 with the bistable insertion mechanism 212 being in the first stable state 212a and with the right side showing the second half of the medical device 200 with the bistable insertion mechanism 212 being in the second stable state 212b.

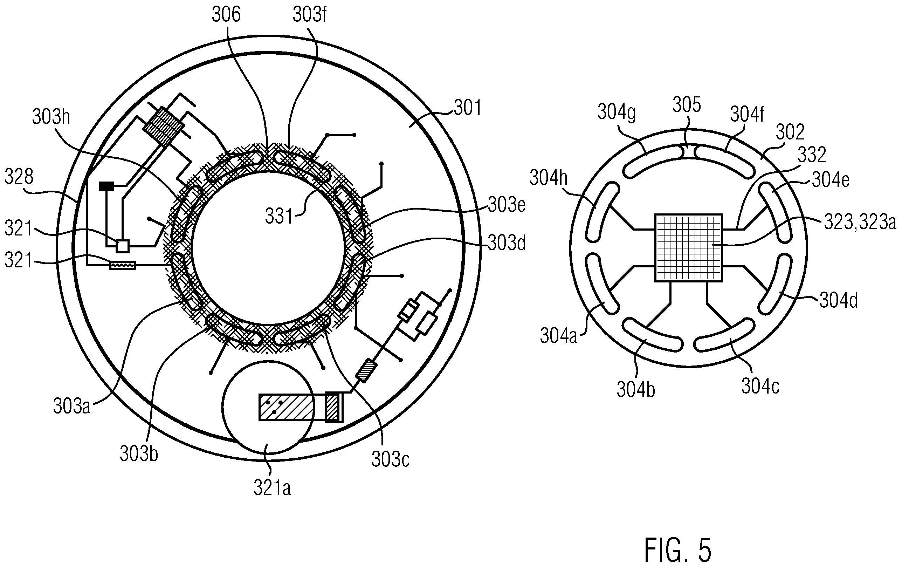

Fig. 5 exemplary shows two possible further exemplary printed circuit board assemblies; a first printed circuit board assembly 301 and a second printed circuit board assembly 302. Said printed circuit board assemblies 301, 302 can be implemented in any of the above and herein described medical devices, e.g. in medical device 100 or in medical device 200.

The printed circuit board assemblies 301, 302 can be identical or similar to the printed circuit board assemblies 130, 122 of Fig. 3 in at least the following ways.

The first printed circuit board assembly 301 can comprise electronic components 321 that can be identical or similar to the electronic components 121 of printed circuit board assembly 130, e.g. the battery 321a can be identical or similar to the battery 121a. Also the first printed circuit board assembly 301 can comprise a plurality of conductors 328, e.g. conductive traces or tracks or paths or inter-integrated circuit (IIC) components that can be identical or similar to the conductors 128 of the printed circuit board assembly 130.

Also, the shape and size of the first printed circuit board assembly 301, including the opening 331, can be identical or similar to the shape and size of the first printed circuit board assembly 130 and its opening 131.

Similarly, the shape and size of the second printed circuit board assembly 302 can be identical or similar to the shape and size of the second printed circuit board assembly 122 of Fig. 3. Similar to the second printed circuit board assembly 122, the second printed circuit board assembly 302 can further comprise one or more electronic components, e.g. on the other side of the printed circuit board assembly 122 that is not visible in Fig. 5. The reference sign 132 exemplary marks possible exemplary conductors or conductive paths on the shown visible side of the second printed circuit board assembly 302 that can be identical or similar to the exemplary conductors or conductive paths 132 of the printed circuit board assembly 122.

The second printed circuit board assembly 302 is coupled, in particular mechanically coupled and/or electrically coupled, to an exemplary analyte sensor 323 with an exemplary array 323a of microneedle analyte sensors, wherein said analyte sensor 323 and the exemplary array 323a of microneedle analyte sensors can be identical or similar to the analyte sensor 123 and the exemplary array 123a of microneedle analyte sensors of Fig. 3.

In contrast to the first printed circuit board 130 of Fig. 3, the first printed circuit board assembly 302 now exemplary comprises eight electrical contact areas 303a, 303b, 303c, 303d, 303e, 303f, 303g, 303h that are circularly arranged around the opening 331.

Furthermore, instead of covering each of the electrical contact areas 303a, 303b, 303c, 303d, 303e, 303f, 303g, 303h with eight separate electrically conductive adhesive transfer tapes or electrically conductive adhesive transfer tape pads, the electrical contact areas 303a, 303b, 303c, 303d, 303e, 303f, 303g, 303h are covered by a single ring-shaped anisotropic electrically conductive adhesive tape 306.

In contrast to the second printed circuit board assembly 122 of Fig. 3, the second printed circuit board assembly 302 comprises eight electrical contact areas 304a, 304b, 304c, 304d, 304e, 304f, 304g, 304h that are exemplary arranged at the periphery of the second printed circuit board assembly 302 and that correspond to the electrical contact areas 303a, 303b, 303c, 303d, 303e, 303f, 303g, 303h of the first printed circuit board assembly 301.

Furthermore, the second printed circuit board assembly 302 comprises two exemplary electrical contact areas 304f, 304g that are bridged, e.g. via jumper or solder bridge 305, thereby forming a single larger contact area that comprises the contact areas 304f, 304g and the bridge 305.

An electrical connection between the first printed circuit board assembly 301 and the second printed circuit board assembly 302 can then again be established as previously described, when the analyte sensor 323 is in its advanced position, i.e. when the bistable insertion mechanism of the medical device transitions from its first stable state to its second stable state.

It is further noted that it is possible that, instead of applying a single ring-shaped anisotropic electrically conductive adhesive tape 306 on the electrical contact areas 303a, 303b, 303c, 303d, 303e, 303f, 303g, 303h of the first printed circuit board assembly 301, single ring-shaped anisotropic electrically conductive adhesive tape could be applied onto the 304a, 304b, 304c, 304d, 304e, 304f, 304g, 304h of the second printed circuit board assembly 302.

The number and arrangement of the electrical contact areas of the printed circuit board assemblies 301, 302 and the number of bridged electrical contact areas are exemplary only.

Furthermore, the exemplary illustrated concave banana shapes of the electrical contact areas 303a, 303b, 303c, 303d, 303e, 303f, 303g, 303h, 304a, 304b, 304c, 304d, 304e, 304f, 304g, 304h are only exemplary and other shapes, e.g. rectangular or elliptic or circular shapes, could be implemented.

Followed by Fig. 1, Fig. 2, Fig. 3, Fig. 4 and Fig. 5, wherein the reference signs denote the following exemplary aspects or features of a herein described exemplary medical device.
100 Exemplary medical device, exemplary wearable medical device, e.g. minimally invasive wearable CGM patch device
100a Exemplary side view of medical device
100b Exemplary view onto the bottom surface of the base part of the medical device
101 Exemplary housing of medical device
102 Exemplary top part of housing
102a Exemplary frame segment, in particular rigid frame segment, of top part of housing
102b Exemplary compliant segment
102bb Exemplary compliant segment, when bistable insertion mechanism is in second stable state
102c Exemplary shuttle
102cc Exemplary displaced shuttle or exemplary displacement position of shuttle, when bistable insertion mechanism is in second stable state
102d, 102e Exemplary joint
103 Exemplary base part of housing
103a Exemplary bottom surface of base part
103b Exemplary top surface of base part
104 Exemplary skin layer, exemplary skin layer above body tissue, exemplary body tissue
105 Exemplary analyte sensor, exemplary array of microneedle analyte sensors
105a Exemplary retracted position of analyte sensor
105b Exemplary advanced position of analyte sensor
106 Exemplary sealing, e.g. gasket
107 Exemplary first printed circuit board assembly
108 Exemplary central line or central axis of medical device, exemplary axis along which the displacement movement of the shuttle and the analyte sensor occurs
108a Exemplary left side of Fig. 1 or exemplary left side of medical device, exemplary first state of medical device
108b Exemplary right side of Fig. 1 or exemplary right side of medical device, exemplary second state of medical device
109 Exemplary cable, e.g. flat flexible cable
110 Exemplary opening or aperture, exemplary though-opening through-hole or through aperture in base part of housing
111 Exemplary insertable portion of the analyte sensor, exemplary insertable portions of the array of microneedle analyte sensors
111a Exemplary microneedle analyte sensors
112 Exemplary bistable insertion mechanism, exemplary compliant bistable insertion mechanism
112a Exemplary first stable state of bistable insertion mechanism at exemplary first position of bistable insertion mechanism
112b Exemplary second stable state of bistable insertion mechanism at exemplary second position of bistable insertion mechanism
113 Exemplary inserter
114 Exemplary second position of bistable insertion mechanism
115 Exemplary first position of bistable insertion mechanism
116 Exemplary displacement path or displacement distance
117 Exemplary adhesive element or adhesive layer, e.g. double-sided adhesive element or double-sided adhesive layer
118 Exemplary removable layer
119 Exemplary gas-permeable membrane
120 Exemplary latch of removable layer
121 Exemplary electronic components on first printed circuit board assembly
121a Exemplary power source, e.g. battery
122 Exemplary second printed circuit board assembly
123 Exemplary analyte sensor, exemplary array 123a of microneedle analyte sensors
124 Exemplary force, exemplary force vector, for triggering transition of bistable insertion mechanism from its first stable state to its second stable state
125 Exemplary radial axis of base part 103
125a Exemplary left side of view 100b
125b Exemplary right side of view 100b
126 Exemplary overlap
126a, 126b, 126c, 126d Exemplary electrical contact areas of first printed circuit board assembly
127a, 127b, 127c, 127d Exemplary electrically conductive adhesive tapes, exemplary electrically conductive adhesive tape pads, of first printed circuit board assembly
128 Exemplary conductors, e.g. conductive traces or tracks or paths or inter-integrated circuit (IIC) components
129a, 129b, 129c, 129d Exemplary electrical contact areas of second printed circuit board assembly
130 Exemplary first printed circuit board assembly
131 Exemplary opening, e.g. through-opening
132 Exemplary conductors, e.g. conductive traces or tracks or paths or inter-integrated circuit (IIC) components
200 Exemplary medical device, exemplary wearable medical device, e.g. minimally invasive wearable CGM patch device
201a Exemplary side view of medical device
201 Exemplary housing of medical device
202 Exemplary top part of housing
202a Exemplary frame segment, in particular rigid frame segment, of top part of housing
202b Exemplary compliant segment
202bb Exemplary compliant segment, when bistable insertion mechanism is in second stable state
202d, 202e Exemplary joint
203 Exemplary base part of housing
204 Exemplary skin layer, exemplary skin layer above body tissue, exemplary body tissue
205 Exemplary analyte sensor, exemplary array of microneedle analyte sensors
205a Exemplary retracted position of analyte sensor
205b Exemplary advanced position of analyte sensor
206 Exemplary sealing, e.g. gasket
207 Exemplary first printed circuit board assembly
208 Exemplary central line or central axis of medical device, exemplary axis along which the displacement movement of the shuttle and the analyte sensor occurs
208a Exemplary left side of Fig. 4 or exemplary left side of medical device, exemplary first state of medical device
208b Exemplary right side of Fig. 4 or exemplary right side of medical device, exemplary second state of medical device
210 Exemplary opening or aperture, exemplary though-opening through-hole or through aperture in base part of housing
211 Exemplary insertable portion of the analyte sensor, exemplary insertable portions of the array of microneedle analyte sensors
211a Exemplary microneedle analyte sensors
212 Exemplary bistable insertion mechanism, exemplary compliant bistable insertion mechanism
212a Exemplary first stable state of bistable insertion mechanism at exemplary first position of bistable insertion mechanism
212b Exemplary second stable state of bistable insertion mechanism at exemplary second position of bistable insertion mechanism
213 Exemplary inserter
214 Exemplary second position of bistable insertion mechanism
215 Exemplary first position of bistable insertion mechanism
216 Exemplary displacement path or displacement distance
217 Exemplary second printed circuit board assembly
217b Exemplary displaced second printed circuit board assembly, exemplary displaced position of second printed circuit board assembly
218a, 218b Exemplary electrical contact areas of second printed circuit board assembly, e.g. covered with electrically conductive adhesive tape pads
219a, 219b Exemplary electrical contact areas of second printed circuit board assembly
220 Exemplary cavity in shuttle
221 Exemplary electronic components on first printed circuit board assembly
222 Exemplary electronic components on second printed circuit board assembly
223 Exemplary force, exemplary force vector, for triggering transition of bistable insertion mechanism from its first stable state to its second stable state
301 Exemplary first printed circuit board assembly
302 Exemplary second printed circuit board assembly
303a, 303b, 303c, 303d, 303e, 303f, 303g, 303h Exemplary electrical contact areas
304a, 304b, 304c, 304d, 304e, 304f, 304g, 304h Exemplary electrical contact areas
305 Exemplary bridge, e.g. jumper or solder bridge
306 Exemplary anisotropic electrically conductive adhesive tape
321 Exemplary electronic components on first printed circuit board assembly
321a Exemplary power source, e.g. battery
323 Exemplary analyte sensor, exemplary array 323a of microneedle analyte sensors
328 Exemplary conductors, e.g. conductive traces or tracks or paths or inter-integrated circuit (IIC) components
331 Exemplary opening, e.g. through-opening
332 Exemplary conductors, e.g. conductive traces or tracks or paths or inter-integrated circuit (IIC) components

## Claims

1. A medical device (100), in particular a wearable medical device (100), for detecting at least one analyte in a body fluid, the medical device (100) comprising:
a housing (101) comprising:
an analyte sensor (105) having an insertable portion (111) adapted for at least partially being insertable into a body tissue of a user;
an inserter (113) comprising a bistable insertion mechanism (112), wherein the bistable mechanism (112) comprises a first stable state (112a) at a first position (115) and a second stable state (112b) at a second position (114),
wherein in the first position (115) of the bistable insertion mechanism (112), the analyte sensor (105) is positioned in a retracted position (105a), and wherein in the second position (114) of the bistable insertion mechanism (112), the analyte sensor (105) is positioned in an advanced position (105b),
and wherein the bistable insertion mechanism (112) is configured for advancing the analyte sensor (105) from the retracted position (105a) to the advanced position (105b) during a transition from the bistable insertion mechanism (112) from the first position (115) to the second position (114), whereby the insertable portion (111) of the analyte sensor (105) can be inserted into a body tissue of a user at least partially.

2. The medical device (100) according to the preceding claim, wherein the analyte sensor (105) comprises an array of microneedle analyte sensors.

3. The medical device (100) according to one of the preceding claims, wherein the analyte sensor (105) is directly coupled to the bistable insertion mechanism (112) or wherein the analyte sensor in the retracted position has no physical contact with the bistable insertion mechanism.

4. The medical device (100) according to one of the preceding claims, wherein the bistable insertion mechanism (112) is a compliant bistable insertion mechanism.

5. The medical device (100) according to one of the preceding claims, wherein the inserter (113) comprising the bistable insertion mechanism is integrated into the housing (101) of the medical device (100).

6. The medical device (100) according to the preceding claim, wherein the housing (101) comprises a top part (102) and a base part (103) with an opening (110), said opening (110) being configured for receiving the analyte sensor (105) in the advanced position, and wherein the inserter (113) comprising the bistable insertion mechanism is integrated in the top part of the housing (102).

7. The medical device (100) according to the preceding claim, wherein the inserter (113) comprising the bistable insertion mechanism (112) is integrated in the top part (102) of the housing (101), and wherein the bistable insertion mechanism (112) is formed by a shuttle (102c), at least one compliant segment (102b) and at least one frame segment (102a), wherein the shuttle (102c) is coupled to the at least one frame segment (102a) via the at least one compliant segment (102b).

8. The medical device (100) according to one of the preceding claims 6 or 7, wherein the base part (103) of the housing (101) comprises at least one adhesive element, in particular a double-sided adhesive element, wherein the at least one adhesive element is configured for providing a sealing for the housing (101) and/or wherein the at least one adhesive element is configured for providing a fixation of the analyte sensor (105) in the advanced position of the analyte sensor; or wherein the analyte sensor (105) comprises at least one adhesive element or wherein the analyte sensor (105) is coupled to at least one adhesive element.

9. The medical device (100, 200) according to one of the preceding claims, further comprising a first printed circuit board assembly (207) and a second printed circuit board assembly (217), wherein the first printed circuit board assembly (207) comprises at least one electronic component, and wherein the second printed circuit board assembly (217) comprises at least one electronic component, and wherein the analyte sensor (205) is coupled to the second printed circuit board assembly (217), and wherein the first printed circuit board assembly (207) and the second printed circuit board assembly (217) are electrically connected to each other via a cable (109) and/or wherein the first printed circuit board assembly (207) and the second printed circuit board assembly (217) comprise one or electrical contact areas that are configured for establishing an electrical connection between the first printed circuit board assembly (207) and the second printed circuit board assembly (217) when the analyte sensor is in the advanced position.

10. The medical device (100, 200) according to the preceding claim, wherein the first printed circuit board assembly (207) and the second printed circuit board assembly (217) comprise one or electrical contact areas that are configured for establishing an electrical connection between the first printed circuit board assembly (207) and the second printed circuit board assembly (217) when the analyte sensor (205) is in the advanced position, and the wherein medical device (100, 200) is configured for being electronically activated, when said electrical connection between the first printed circuit board assembly (207) and the second printed circuit board assembly (217) is established via said electrical contact areas.

11. The medical device (100, 200) according to the preceding claim, wherein at least some of the one or more electrical contact areas comprise an electrically conductive adhesive tape.

12. The medical device (100, 200) according to one of the preceding claims 10 or 11, wherein the first printed circuit board assembly (207) and/or the second printed circuit board assembly (217) comprises at least two electrical contact areas that are bridged.

13. The medical device (100, 200) according to one of the preceding claims 6 to 12, further comprising a removable layer covering the base part (103, 203) and wherein the layer comprises or is coupled to a gas-permeable membrane that covers the opening (110, 210) of the base part (103, 203).

14. A method of using the medical device (100, 200) according to one of the preceding claims, the method comprising:
providing the medical device (100, 200), wherein the bistable insertion mechanism (112, 212) is in the first stable state at the first position and in which the analyte sensor (105, 205) is positioned in the retracted position;
placing the medical device (100, 200) against a skin layer of a user;
applying a force, e.g. a pushing force, to the bistable insertion mechanism (112, 212) to bring the bistable insertion mechanism into its second stable state in the second position, thereby advancing the analyte sensor (105, 205) from the retracted position to the advanced position to at least partially insert the insertable portion (111, 211) of the analyte sensor (105, 205) into a body tissue of the user.

15. Method for assembling a medical device (100, 200) according to any one of the preceding medical device claims, wherein the method comprises:
providing a first part, e.g. the top part (102, 202), of the housing (101) comprising the inserter (113) that comprises the bistable insertion mechanism;
providing a second part, e.g. the base part (103, 203), of the housing (101, 201);
mounting the first printed circuit board assembly (107, 207) comprising at least one electronic component to the second part of the housing (101, 201);
securing the first part of the housing and the second part of the housing (101, 201) to each other.
